# EUROPEAN PATENT SPECIFICATION

(11) **EP 0 406 386 B1**
(45) Date of publication and mention of the grant of the patent: **03.01.1996**
(21) Application number: 90901653.7
(22) Date of filing: 10.01.1990
(51) Int. Cl.: C07C 251/58, C07C 249/12, C07D 213/58, C07D 413/12, C07D 401/12

(54) **IMPROVED PROCESS FOR THE PREPARATION OF AMIDOXIME DERIVATIVES**
VERFAHREN ZUR HERSTELLUNG VON AMIDOXIM-DERIVATEN
PROCEDE AMELIORE POUR LA PREPARATION DE DERIVES D'AMIDOXIME

(30) Priority: 10.01.1989 HU 7089
(43) Date of publication of application: 09.01.1991
(73) Proprietor: CHINOIN Gyogyszer és Vegyészeti Termékek Gyára RT., H-1045 Budapest IV (HU)
(72) Inventor: BERTOK, Béla, H-1221 Budapest (HU); SZEKELY, István, H-2120 Dunakeszi (HU); THURNER, Angelika, H-1116 Budapest (HU); SOMFAI, Eva, H-1014 Budapest (HU); BOTAR, Sándor, H-1221 Budapest (HU); GAJARY, Antal, H-1021 Budapest (HU); TAKACS, Kálmán, H-1088 Budapest (HU); NAGY, Lajos, H-2000 Szentendre (HU)
(74) Representative: Patentanwälte Beetz - Timpe - Siegfried Schmitt-Fumian - Mayr
(86) International application number: PCT/HU90/00003
(87) International publication number: WO 90/08131

(56) References cited:
- EP-A- 0 023 560
- EP-A- 0 121 701
- CH-A- 0 634 828
- DE-A- 3 620 166
- GB-A- 1 582 029
- US-A- 4 187 220
- US-A- 4 308 399

## Description

This invention relates to an improved process for the preparation of amidoxime derivatives of the general formula I, 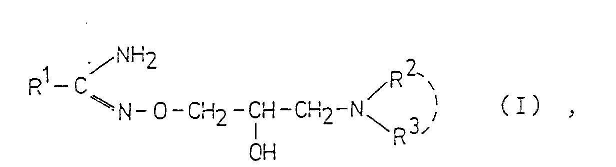 wherein are:
- R¹: a C₂₋₁₅ group, which may be an unsaturated and/or cyclic alkyl or aralkyl group or an optionally substituted and/or condensed aromatic and/or heteroaromatic group;
- R: hydrogen, an optionally substituted straight or branched chain or cyclic and/or unsaturated C₁₋₇ alkyl or aromatic group,
and
- R³: an optionally substituted straight or branched chain or cyclic and/or unsaturated C₁₋₇ alkyl or aromatic group, wherein
R and R³ may form together with the adjacent nitrogen atom a 5- to 8-membered ring optionally containing other heteroatom(s), and their salts.

It is known that several amidoxime derivatives of the general formula I are useful for the treatment of diabetic angiopathy which is practically unique up to the present. Other amidoxime derivatives show antihypertensive action, too, and eventually, an alpha-blocking effect may also be observed (GB-A-1 582 029; US-A-4 187 220 and 4 308 399). The possibility of using these compounds as therapeutic drugs requires an economical preparation which can be carried out on an industrial scale, too.

It is known that O-substitued derivatives of oximes are usually prepared by employing alkylating agents (Houben Weyl Vol. X/4 (1968) pages 217 to 220.

These have been known two process variants for the preparation of amidoxime derivatives of the above-indicated general formula I and their salts :

The first variant comprises the following steps :
(A) reaction of an amidoxime of the general formula II, 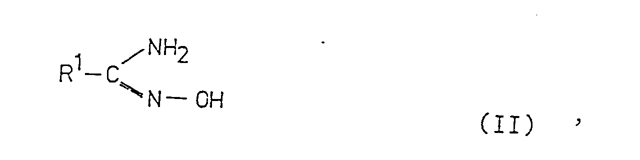 wherein R¹ is as defined above,
   with an alkali metal hydroxide or an alkali metal alkoxide in the presence of an organic solvent,
(B) reaction of the amidoxime complex obtained in step A with an amino compound under SN2 reaction conditions at a temperature between 0 and 100 °C,
   and
(C) isolating the obtained amidoxime derivative of formula I,
(D) and optionally
   converting the amidoxime derivative of formula I with one or more acids into the corresponding salt or salt mixtures, respectively, and optionally converting the salts into the free base, and optionally converting the free base with another acid into the corresponding salt.
and their salts

The second variant comprises the following steps :
(A) reaction of an amidoxime of the general formula II, 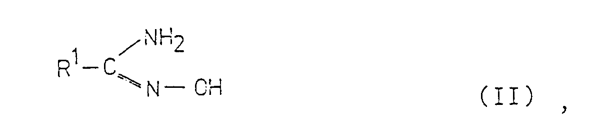 wherein R¹ is as defined above,
   with an alkali metal hydroxide or an alkali metal alkoxide in the presence of an organic solvent,
(B) reaction of the amidoxime complex obtained in step A
   (B1) with 1-halo-2-hydroxy-3-propanamine of the general formula III 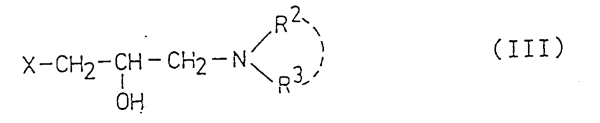
   (B2) and/or an epoxyamine of the general formula IV 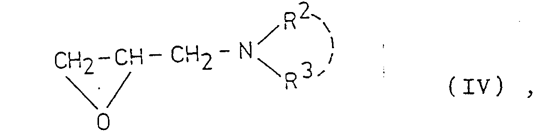 wherein R and R³ are as defined above, and X is a halogen atom,
   under SN2 reaction conditions at a temperature between 0 and 100 °C,
   and
(C) isolating the obtained amidoxime derivative of formula I,
(D) and optionally
   converting the amidoxime derivative of formula I with one or more acids into the corresponding salt or salt mixtures, respectively, and optionally converting the salts into the free base, and optionally converting the free base with another acid into the corresponding salt.

The above process variants have been known from GB-A- 1 582 029.

Protic solvents such as water, methanol, ethanol or mixtures of water with a water-immiscible solvent, e.g. benzene, were used as solvents in these reactions. The final products were isolated by extraction (eventually after evaporation), the extract was washed several times with a concentrated alkaline solution, and after solvent change it was acidified with alcoholic hydrochloric acid and carefully crystallized. The hydrochlorides of the products were obtained in this way in yields between 6 and 50 %.

On reproduction and scale increase of these reactions it has surprisingly been found that the amidoximes of the general formula II could not completely be transformed. After a conversion of about 60 to 70 %, the reaction stopped, and both an increase in the reaction temperature and the use of an excess of reactant enhanced the amount of tarry by-products which inhibited the isolation of the desired product.

It is known from the literature (J. Am. Chem. Soc. 80 (1958) 1257 ; Appl. Polym. Sci. 11, (1967) 145-8 ; and J. Chem. Soc. 1950 2257-2272) that amines of the general formula III are unstable: on standing they are reversibly transformed into a cyclic 3-hydroxyazetidine salt of the general formula V 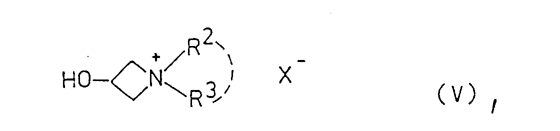 wherein R and R³ are as defined above, or dimerize to a dioxane derivative of the general formula VI 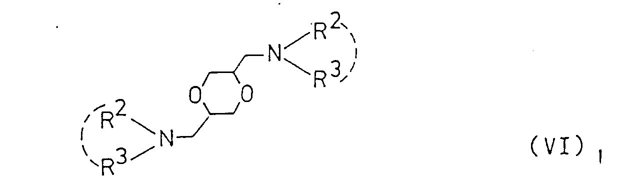 wherein R and R³ are as defined above.

Concerning the synthesis, the former transformation does not represent any drawback since amidoximes can be reacted with salts of the general formula V in the presence of a base. However, the latter transformation is irreversible which can cause a significant substance loss in the synthesis.

One basis of the present invention consists in the recognition that the compounds of the general formulae III, IV or V, respectively, react also with water, alcohols and acids to give by-products of the general formula IX 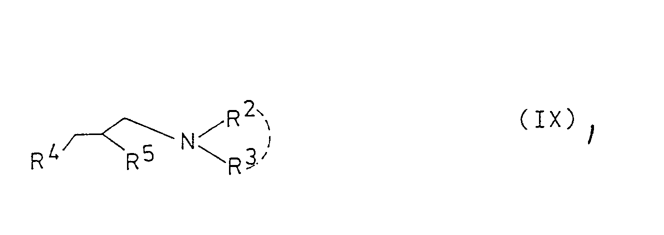 wherein
- R⁴ and R⁵: stand for hydroxy, C₁₋₄ alkoxy, C₂₋₈ alkanoyl or an acid residue.

Depending on the dimensions of the R⁴ and R⁵ groups, particularly in the case of hydroxy or lower alkoxy groups, polymerization can also occur to a significant extent wich can lead e.g. to compounds of the general formula XI

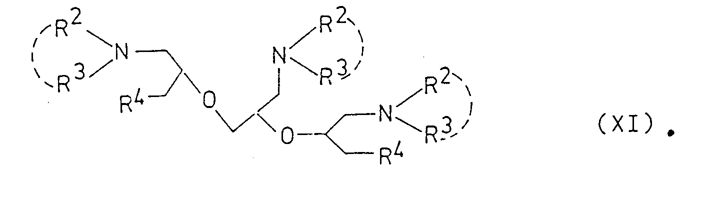

The present invention is based on the further finding that the appearance of these decomposition products is responsible for the "stopping" of the coupling reaction. The appearance of the decomposition products is presumably favourable for SN1 reactions and therefore results in a protic system promoting a further decomposition and inhibiting the reaction of the amidoxime. It has been found by using model reactions that the reactants of the general formulae III, IV and V are very unstable under the conditions described up to the present, and the side reactions are promoted either by basic or acidic catalysis. Thus, in the case of the epoxide of general formula IV the base catalysis favours a terminal substitution whereas an acid catalysis promotes the substitution on the C-2 carbon atom. In the coupling reaction with the amidoxime, the epoxide of the general formula IV is completely decomposed within 2 to 4 hours. Based on the above recognitions it was the first aim above all to find a reaction medium or reaction conditions, respectively , wherein the reactants are not decomposed, and the selective coupling of the amidoxime of general formula II and the formation of the final product are promoted and accelerated either by suppressing the formation of the detected contaminants or by separating the final product from these contaminants eventually formed in small amounts.

Thus, it is the object of the present invention to provide processes for the preparation of amidoximes of the general formula I which are free from the above disadvantages and which can be carried out on an industrial scale.

The above object is achieved according to the independent claims. The dependent claims relate to prefered embodiments. The process of the present invention for the preparation of amidoxime derivatives of the general formula I according to the first variant. is characterized in that
- dimethyl formamide or 1,3-dimethyl-2-imidazolidinone is used as organic solvent in step A,
   and
- a compound of the formula V, 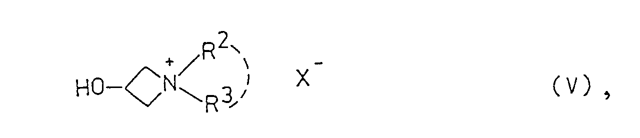 wherein R and R³ are as defined above, and X is a halogen atom,
is used as amino compound in step B.

The process of the present invention for preparing the amidoxime derivatives of the general formula I according to the above-indicated second variant is characterized in that dimethyl formamide or 1,3-dimethyl-2-imidazolidinone is used as organic solvent in step A.

The reaction in step A is preferably carried out in the presence of a proton source. The amidoxime complex thus obtained is preferably brought to reaction without isolation with the amine of the general formulae III and/or IV and/or V, suitably in the presence of a metal salt catalyst. Then, if desired, the thus formed product of the general formula I is selectively separated from the by-products and/or converted with acids into salts, if desired, to mixed salts, crystallized or, if desired, converted into the base and/or again transformed into a salt by using another acid.

According to a preferred embodiment of the processes of the invention, the reactants of the general formulae III, IV and V are used in an excess of 0.05 to 0.15 equivalent, and optionally, a catalyst is employed.

It is suitable to use dimethylformamide (DMF) or 1,3-dimethyl-2-imidazolidinone (DMI) with a water content of 1 % or less which are free from contaminations arising e.g. from a decomposition since otherwise the yield may be deteriorated, or an undesired transformation of DMF or DMI may be induced.

The formation and deliberate preparation, respectively, of the complex are substantial factors of the present invention. A very significant amount of precipitate is observed if the reaction is carried out in dimethylformamide at 50 °C using an alkali hydroxide as base. This precipitate gradually disappears as the reaction proceeds. It is not necessary to separate the complex from the mixture. For improving the solubility conditions and ensuring the proton transfer it is preferred to add a proton source, preferably tertiary butanol, to the mixture whereby the yield is increased by 3 to 5 %, and the reaction time is shortened. The complex is then brought to further reaction, preferably at 30 to 75 °C.

It has been found that the purity of the starting amines of general formulae III, IV or V, respectively, is important for the course of the reaction. All these three compounds excellently react in a pure state whereas purification and storage of these reactants can be carried out only with difficulties on an industrial scale because of their high reactivity. Therefore, a simple method for preparing these products was developed in connection with the solution of the present invention.

It has been found that it is suitable to allow the amidoxime complex prepared according to the present invention to react with an amine of the general formulae III, IV or V, respectively, which has been prepared by reaction of epichlorohydrin with an amine of the general formula VII 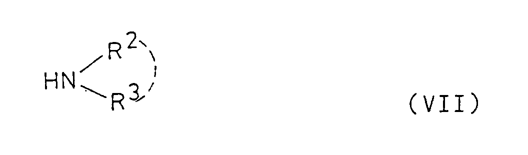 in the presence of tertiary butanol taken in a ratio of 1:0.8 to 1:1.2, based on the mass of the amine.

Preferably, the amines of formulae III, IV or V are allowed to react with the complex without isolation from the reaction mixture after their preparation. In this way, the amidoxime derivative of the general formula I can be prepared in a good yield in the same apparatus "one-shot" reaction).

However, the reaction lasts for a longer time owing to the inhibiting effect of the decomposition products discussed above. In these cases the use of the catalysts mentioned above is particularly preferred.

Thus, according to the present invention, the target compounds can be prepared with a good efficiency, and at the same time, side reactions can be suppressed. However, as the product contains some amount of contaminating substances, the invention further relates to a selective separation of the final product from the accompanying undesired substances.

For the selective separation of the product the reaction mixture is optionally diluted with a solvent, neutralized with an acid at 40 to 70 °C optionally after filtration then, optionally after a repeated filtration; the mixture is then acidified, and the salts and contaminants (e.g. the catalyst) precipitated at a pH value of 4 to 5 at 50 °C or at a pH value of 2 to 3 at 70 °C are separated from the reaction mixture, and subsequently, the salt of the amidoxime derivative of general formula I formed with an acid is crystallized by adjusting the pH of the reaction mixture to 1 to 3. Hydrochloric acid is preferably used for the acidification.

The process according to the invention is more simple than those known up to the present, and the purity of the substances obtained as crude products is considerably higher. From the operational point of view, it is easy to carry out the processes on an industrial scale. The yields are excellent (75 to 97 %) and can further be improved by working up the mother liquors of the crystallization. After evaporation and alkaline extraction of the mother liquor the solution can again be crystallized by acidification as described above.

By using the process according to the present invention O-(2-hydroxy-3-piperidino-1-propyl) nicotinic acid amidoxime base could be successfully isolated in a pure, crystalline state, m.p. 70-73 °C, which had not been described up to the present. The most important physico-chemical characteristics of this substance are as follows.

IR spectrum (KBr): ν-O-C=N 1642 cm⁻¹
¹H-NMR spectrum (CDC1₃, δ ppm): 1.48 m, (6H), CH₂-piperidine; 2.42 m, (6H), 3×CH₂N; 3.36, br, (1H), CH-O; 4.08 m, (3H), 1-CH₂, OH; 5.2, br, (2H), NH₂; 7.30 m (1H), pyridine-5'; 7.92 m, 4', 8,62 m (1H), 6', 8,88 m, (1H), 2'.
1 g of this substance dissolved in 10 ml of concentrated sulfuric acid gives a yellowish homogeneous solution.

Similarly, it has been successful to obtain in a very pure state O-(2-hydroxy-3-piperidino-1-propyl) nicotinic acid amidoxime hydrochloride and hydrobromide. The hydrobromide had not been described up to the present; the hydrochloride has been disclosed, however only in an unsuitable purity. The spectroscopic characteristics of these new substances are as follows.
UV spectrum: λₘₐₓ 274.237 nm
IR spectrum (KBr): ν -O-C=N 1649 cm⁻¹
¹H-NMR spectrum (DMSO-d₆, δ ppm): 1.80, br, (6H), CH₂--piperidine; 2.65-3.75 m, (6H 3×CH₂-N); 4.00 m, (2H), 1-CH₂; 4.40 m (1H), CH-O; 7.00, br, (4H), 2×NH⁺, NH₂; 8.00 dd, (1H), pyridine-5'; 8.75 m, (1H), 4'; 8.95 m, (1H), 6'; 9.13 d, (1H), 2'; 10.35, br, (1H), OH.

1 g dissolved in 10 ml of concentrated sulfuric acid gives a yellowish homogeneous solution.

The processes of the invention are illustrated in detail by the following Examples.

### Example 1

1.0 g (25 mmol) of powdered sodium hydroxide and 4.7 ml (50 mmol) of tert-butanol are added to a solution of 6.9 g (50 mmol) of nicotinic acid amidoxime in 50 ml of pure, dry DMF. To the resulting suspension 7.8 g (55 mmol) of redistilled N-(2,3-epoxypropyl)piperidine (J. Am. Chem. Soc. 80 (1958) 1257-9 are added at 50 °C and stirred at 70 °C. The course of the reaction is observed by thin layer chromatography (TLC). After complete termination of the reaction the pH of the solution is adjusted to 6, and after clarifying and filtering, the filtrate is acidified to pH 2.5 and crystallized. The pale yellow crystalline precipitate is filtered to give O-(2--hydroxy-3-piperidino-1-propyl) nicotinic acid amidoxime dihydrochloride in a yield of 16.5 g (94 %), m.p. 202-204 °C, after recrystallization from ethanol. The first fraction is 14.9 g of a pale yellow product which is of 99.1 % purity, based on the displacement method or on the determination of C1⁻, and of 99.2 % purity on spectrophotometric and nitrogen determination, m.p. 202-204 °C. The yield is 85 %.

UV spectrum: λₘₐₓ 274.237 nm
IR spectrum (KBr): ν -O-C=N 1649 cm⁻¹
¹H-NMR spectrum (DMSO-d₆, δppm): 1.80, br, (6H), CH₂-piperidine; 2.65-375 m, (6H, 3×CH₂-N); 4.00 m, (2H), 1-CH₂; 4.40 m, (1H), CH-O; 7.00, br, (4H) 2×NH⁺, NH₂; 8.00 dd, (1H), pyridine-5'; 8.75 m, (1H), 4'; 8.95 m, (1H), 6'; 9.13 d, (1H), 2'; 10,35, br. (1H), OH.
1 g of the product dissolved in 10 ml of concentrated sulfuric acid gives a yellowish homogeneous solution.

### Example 2

435 ml of dry tert-butanol and 676 g (4.785 mol) of N--(2,3-epoxypropyl)-piperidine are added to a mixture containing 596 g (4.35 mol) of nicotinic acid amidoxime, 4350 ml of pure, dry DMF and 100 g (2.5 mol) of sodium hydroxide. The pale brown suspension is stirred at 65 °C for 3 hours. The course of the reaction is observed as described in Example 1. After acidifying the mixture to pH 6 it is clarified and filtered, and the dark yellow solution is adjusted to pH 2.5. The pale yellow crystalline precipitate is filtered to give 1480 g of O-(2-hydroxy-3-piperidino-1-propyl) nicotinic acid amidoxime dihydrochloride in a purity of 95 %. The yield is 92 %. After recrystallization the total yield together with the second and third fractions amounts to 88 %, calculated for the starting substances. The purity of the crystallized product is 99.5 %, m.p. 202-204 °C. The product is identical to that of Example 1.

### Example 3

After mixing 41.3 g of nicotinic acid amidoxime, 750 ml of dry DMF and 6 g of NaOH, 46.6 g of N-(2,3-epoxypropyl)-piperidine are added to the complex obtained, and the mixture is allowed to react at 65 °C for 4 hours.The orange red thick suspension is transformed into a pale brown solution during the course of the reaction. After distilling off 400 ml of DMF from the solution during 90 minutes the mixture is cooled to room temperature and diluted with 500 ml of dry isopropanol. After adjusting the pH of the solution to 6 to 7 and then filtering off the contaminations, the pH of the pale yellow solution is adjusted to 2.5 by adding hydrochloric acid and left standing to slowly crystallize. 81 g of butter-coloured crystalline hydrochloride are obtained, m.p. 202-205 °C. This product is analytically identical to those described in the preceeding Examples. From the mother liquor 14.5 g of a pale yellow crystalline product are obtained as second fraction, m.p. 195-200 °C. The total yield amounts to 90.6 %.

### Example 4

By treating O-(2-hydroxy-3-piperidino-1--propyl) nicotinic acid amidoxime dihydrochloride with sodium hydroxide solution the O-(2-hydroxy-3-piperidino-1-propyl) nicotinic acid amidoxime base is obtained and isolated as a white crystalline substance in a pure state, m.p. 70-73 °C.
IR spectrum (KBr): ν -O-C=N- 1642 cm⁻¹
¹H-NMR spectrum (CDC1₃, δ ppm):1,48 m, (6H), CH₂-piperidine; 2,42 m, (6H), 3×CH₂N; 3,36, br, (1H), CH-O; 4,08 m, (3H), 1-CH₂, CH; 5,2 br, (2H), NH₂; 7,30m, (1H), piridine 5'; 7,92 m, (1H); 4'; 8,62 m, (1H), 6'; 8,88 m, (1H), 2'.
1 g of this substance dissolved in 10 ml of concentrated sulfuric acid gives a yellowish homogeneous solution.

### Example 5

O-(2-hydroxy-5-piperidinol-1-propyl) nicotinic acid amidoxime is prepared as described in Example 1, except that the acidification is carried out with an ethanolic solution of dry hydrogen bromide to obtain 20.5 g of O-(2-hydroxy-3-piperidino-1-propyl) nicotinic acid amidoxime dihydrobromide in a yield of 93 % (together with the second fraction), m.p. 180-184 °C.

### Example 6

The process described in Example 1 is followed, except that the salt is formed by using isopropanol saturated with dry hydrogen chloride (concentration about 8.5 mmol/ml) to give O-(2-hydroxy-3-piperidino-1-propyl) nicotinic acid amidoxime dihydrochloride in a yield of 95 %.

### Example 7

The process described in Example 1 is followed, except that no tert-butanol is used. The reaction mixture is stirred for 6 hours at 70 °C to give a total yield of 94 %.

### Example 8

The process described in Example 1 is followed, except that no tert-butanol is used, and 1.12 g (10 mmol) of potassium tert-butoxide are employed as base. After stirring the reaction mixture at 70 °C for 3 hours a total yield of 16.67 g (95 %) is obtained.

### Example 9

The process described in Example 1 is followed, except that 70 ml of DMF and 5.0 g (125 mmol) of sodium hydroxide are used and as reactant 11.8 g (55 mol) of 1-chloro-2-hydroxy-3-piperidinopropane hydrochloride ( Monatshefte für Chemie 15, 119) are added. After stirring the reaction mixture at 70 °C for 6 hours the product is obtained in a total yield of 16.7 g (95 %).

### Example 10

The process described in Example 1 is followed, except that 3.2 g (80 mmol) of powdered sodium hydroxide and 7.5 ml of tert-butanol are used and as reactant 9.8 g (55 mmol) of 1,1-pentamethylene-3-hydroxyazetidinium chloride (J. Org. Chem. 33 (2) 523) are added. After stirring the suspension at 70 °C for 3 hours O-(2-hydroxy-3-piperidino-1-propyl) nicotinic acid amidoxime dihydrochloride is obtained in a total yield of 15.8 g (90 %).

### Example 11

The process described in Example 1 is followed, except that 1.0 g (10 mmol) of aluminum oxide is used as catalyst. After recrystallization the product is obtained in a yield of 14.9 g (85 %), m.p. 206-209 °C.

### Example 12

The process described in Example 1 is followed, except that 1.5 g (10 mmol) of stannic oxide are used as catalyst. After recrystallization the product is obtained in a yield of 14.7 g (84 %), m.p. 207-210 °C.

### Example 14

The process described in Example 1 is followed, except that 9.3 g (50 mmol) of 2-amino-5-chlorobenzamidoxime are used as amidoxime component, and after acidification the reaction mixture is evaporated under reduced pressure. The thick oily residue is dissolved in 50 ml of hot isopropanol and left standing to crystallize to give a yield of 15.0 g, m.p. 189-190 °C.

### Example 15

4.7 g (5.5 ml; 55 mmol) of piperidine are added during 20 minutes to a solution of 5.1 g (4.3 ml; 55 mmol) of epichlorohydrin in 5.2 ml of tert-butanol under cooling with water while stirring vigorously. The reaction mixture is stirred at room temperature for 1 hour, and then 50 ml of pure dry DMF, 6.9 g (50 mmol) of nicotinic acid amidoxime and 3.2 g (80 mmol) of powdered sodium hydroxide are added. After stirring the suspension at 70 °C for 12 hours the reaction mixture is worked up as described in Example 1 to give 13.2 g (75 %) of O-(3-amino-2-hydroxypropyl) nicotinic acid amidoxime dihydrochloride.

### Examples 16 to 26

General process for the preparation of O-alkylated acid amidoxime derivatives of the general formula (I).

### Method A

50 mmol of an amidoxime of the general formula II are dissolved in 50 ml of pure dry DMF, and then, 25 mmol of a basic catalyst, 4.7 ml (50 mmol) of tert-butanol and finally 55 mmol of an 1-(2,3-epoxypropyl)-amine of the general formula IV are added.

After stirring at 70 °C until complete termination of the reaction, the mixture is acidified as described in Example 1, and then the product of the general formula I is isolated.

### Method B

The process described under Method A is followed, except that the reaction mixture is acidified and evaporated as described in Example 14, and the product obtained is crystallized from a solvent.

### Method C

The process described under Method A is followed, except that the free base is liberated as described in Example 4 and then is crystallized from a solvent or isolated as an oil.

The results are shown in the following table.

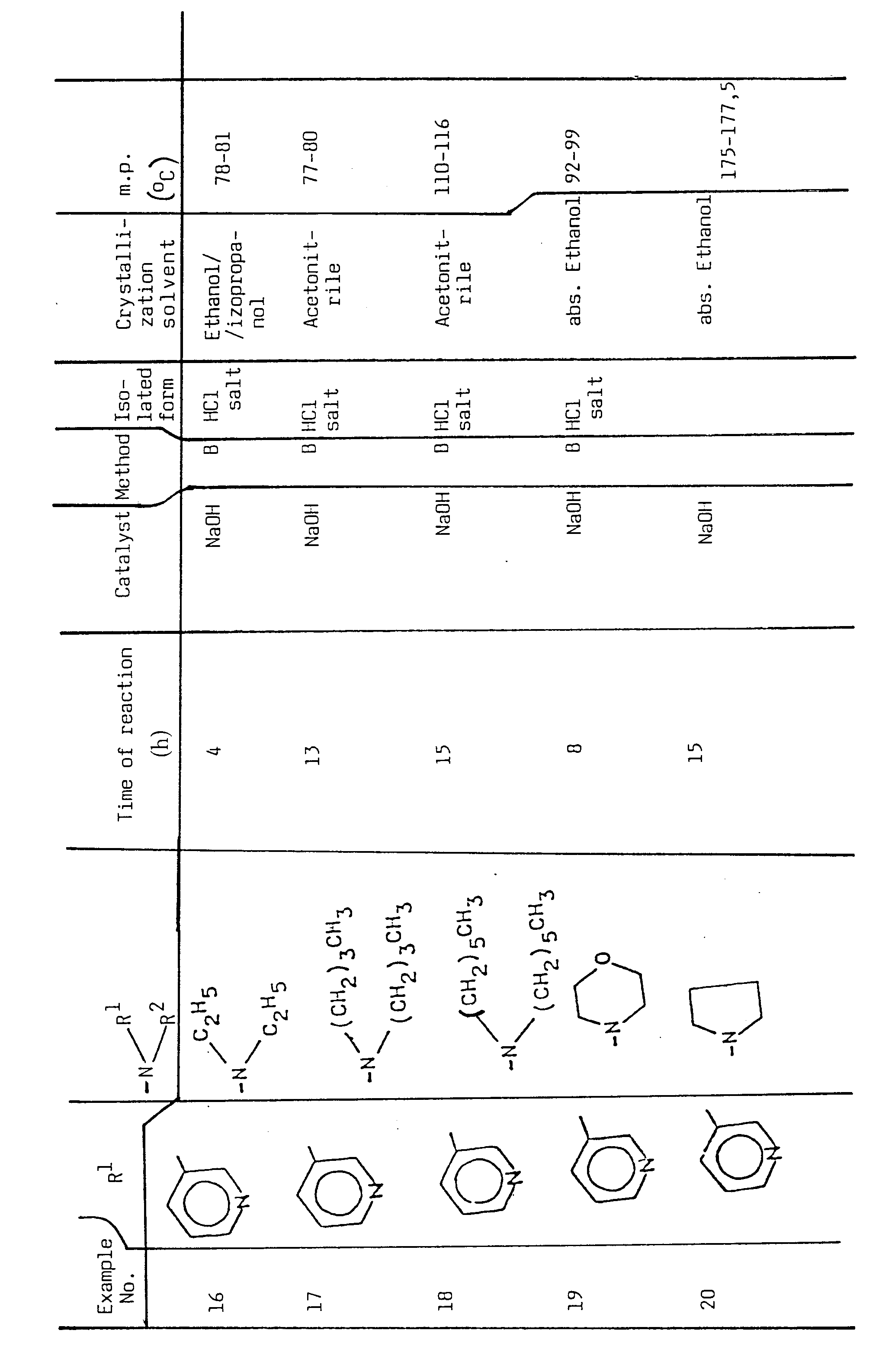

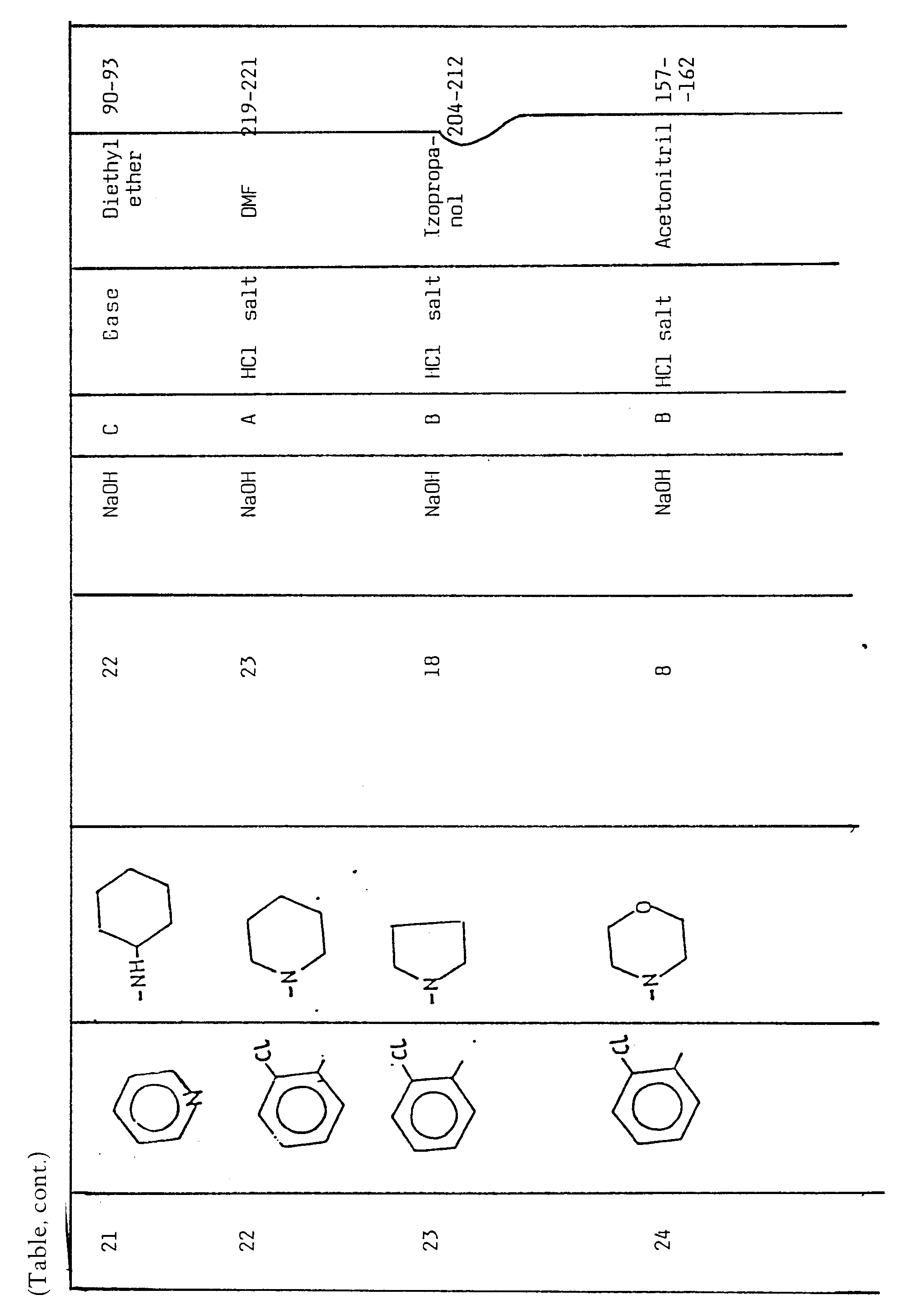

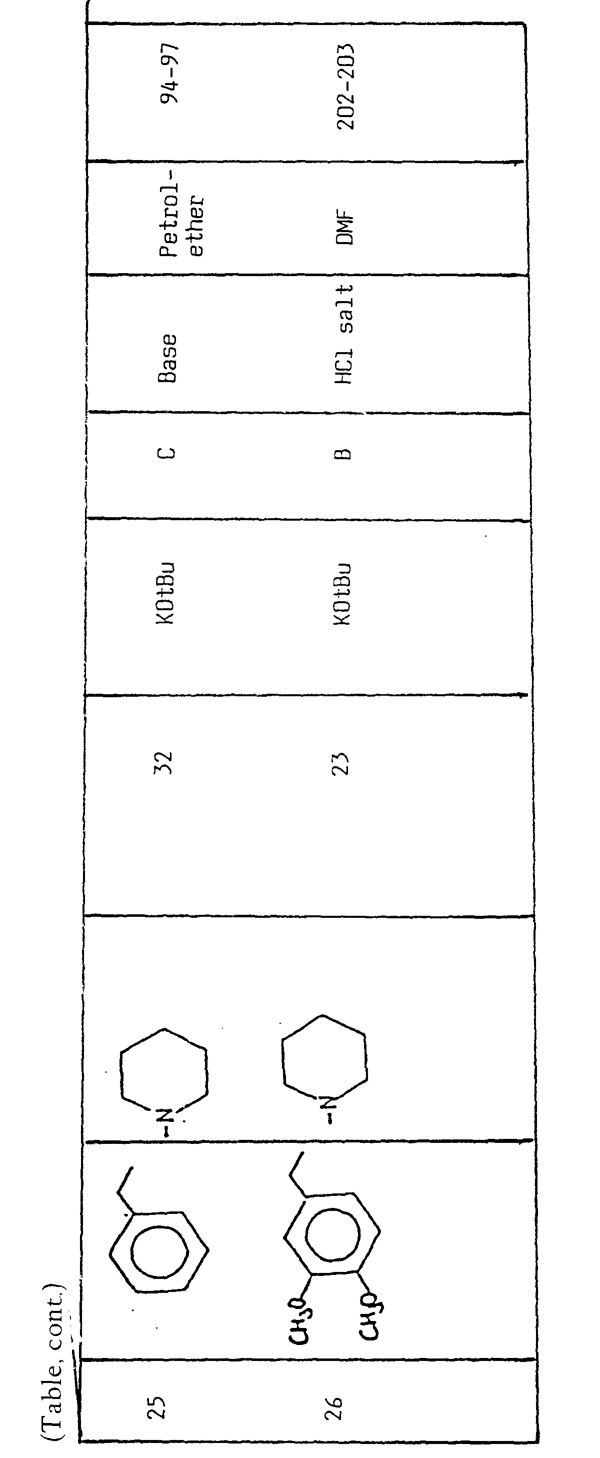

## Claims

1. A process for the preparation of amidoxime derivatives of the general formula I, 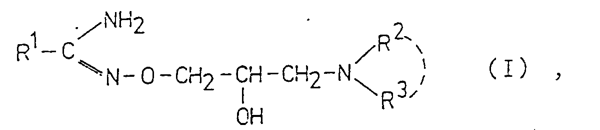 wherein are:
R¹ a C₂₋₁₅ group, which may be an unsaturated and/or cyclic alkyl or aralkyl group or an optionally substituted and/or condensed aromatic and/or heteroaromatic group;
R hydrogen, an optionally substituted straight or branched chain or cyclic and/or unsaturated C₁₋₇ alkyl or aromatic group,
and
R³ an optionally substituted straight or branched chain or cyclic and/or unsaturated C₁₋₇ alkyl or aromatic group, wherein
R and R³ may form together with the adjacent nitrogen atom a 5- to 8-membered ring optionally containing other heteroatom(s),
and their salts
by
(A) reaction of an amidoxime of the general formula II, 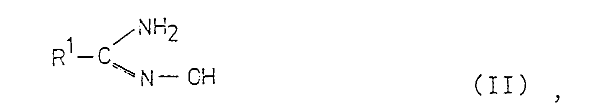
wherein R¹ is as defined above,
with an alkali metal hydroxide or an alkali metal alkoxide in the presence of an organic solvent,
(B) reaction of the amidoxime complex obtained in step A
(B1) with an amine
of the general formula III 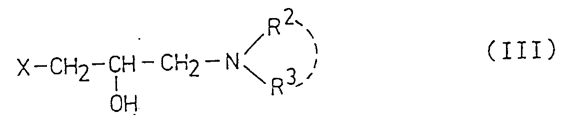
(B2) and/or an amine
of the general formula IV 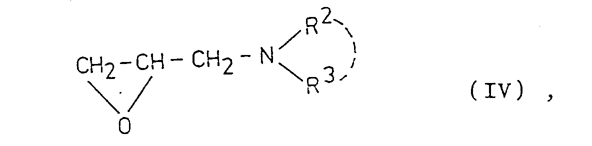 wherein R and R³ are as defined above, and X is a halogen atom,
under SN2 reaction conditions at a temperature between 0 and 100 °C,
and
(C) isolating the obtained amidoxime derivative of formula I,
(D) and optionally
converting the amidoxime derivative of formula I with one or more acids into the corresponding salt or salt mixtures, respectively, and optionally converting the salts into the free base, and optionally converting the free base with another acid into the corresponding salt,
characterized in that
dimethyl formamide or 1,3-dimethyl-2-imidazolidinone is used as organic solvent in step A.

2. A process for the preparation of amidoxime derivatives of the general formula I, 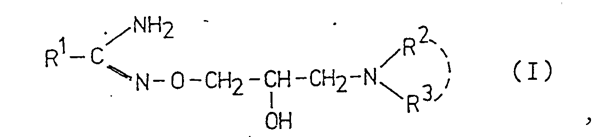 wherein are:
R¹ a C₂₋₁₅ group, which may be an unsaturated and/or cyclic alkyl or aralkyl group or an optionally substituted and/or condensed aromatic and/or heteroaromatic group;
R hydrogen, an optionally substituted straight or branched chain or cyclic and/or unsaturated C₁₋₇ alkyl or aromatic group, and
R³ an optionally substituted straight or branched chain or cyclic and/or unsaturated C₁₋₇ alkyl or aromatic group, wherein
R and R³ may form together with the adjacent nitrogen atom a 5- to 8-membered ring optionally containing other heteroatom(s),
and their salts
by
(A) reaction of an amidoxime of the general formula II, 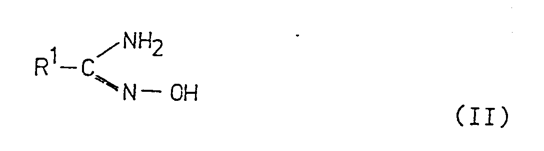
wherein R¹ is as defined above,
with an alkali metal hydroxide or an alkali metal alkoxide in the presence of an organic solvent,
(B) reaction of the amidoxime complex obtained in step A with an amino compound under SN2 reaction conditions at a temperature between 0 and 100 °C,
and
(C) isolating the obtained amidoxime derivative of formula I,
(D) and optionally
converting the amidoxime derivative of formula I with one or more acids into the corresponding salt or salt mixtures, respectively, and optionally converting the salts into the free base, and optionally converting the free base with another acid into the corresponding salt,
characterized in that
- dimethyl formamide or 1,3-dimethyl-2-imidazolidinone is used as organic solvent in step A,
and
- a compound of the formula V, 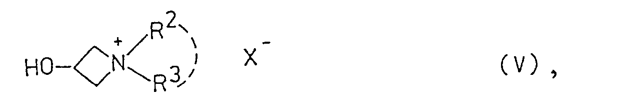 wherein R and R³ are as defined above, and X is a halogen atom,
is used as amino compound in step B.

3. The process according to claim 1 or 2,
characterized in that the reaction in step A is carried out in the presence of a proton source.

4. The process according to claim 3, characterized in that tertiary butanol is used as proton source in step A.

5. The process according to one of claims 1 to 4, characterized in that the amidoxime complex obtained in step A is used in step B without isolation.

6. The process according to one of claims 1 to 5, characterized in that the reaction of the amidoxime complex with the amine in step B is carried out at a temperature of 30 to 75°C.

7. The process according to one of claims 1 to 6, characterized in that the reaction of the amidoxime complex with the amine in step B is carried out in the presence of a metal salt catalyst.

8. The process according to claim 7, characterized in that aluminum oxide, tin oxide, dibutyltin oxide or organic complexes thereof are used as metal salt catalysts.

9. The process according to one of claims 1 to 8, characterized in that the amidoxime derivative of formula I is selectively separated from by-products.

10. The process according to claim 9, characterized by the following steps for the selective separation of the amidoxime derivative of formula I:
- Neutralizing the reaction mixture with an acid at a temperature of 40 to 70 °C, optionally after dilution of the reaction mixture with a solvent and/or after filtration,
- acidifying the obtained mixture,
- adjusting a pH value of 4 to 5 at 50 °C or a pH value of 2 to 3 at 70 °C, and separating the precipitated salts and contaminants from the mixture,
and
- crystallizing the salt of the amidoxime derivative of formula I formed with an acid by adjusting the pH value of the mixture at a value of 1 to 3.

11. The process according to one of claims 3 to 10, characterized in that the amine of formula IV is prepared by reaction of epichlorohydrin of formula VI 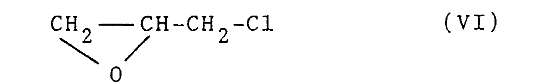 with an amine of the general formula VII, 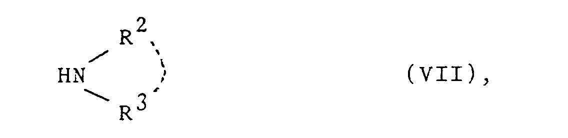
wherein R and R³ are as defined in claim 1,
in the presence of tertiary butanol in a mass proportion of tertiary butanol to amine of formula VII of 1:0.8 to 1:1.2.

## Patentansprüche

1. Verfahren zur Herstellung von Amidoxim-Derivaten der allgemeinen Formel I, 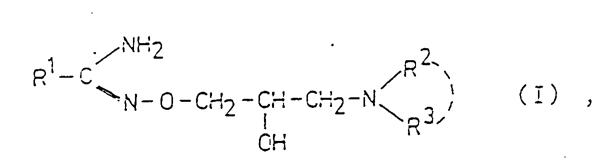 in der bedeuten:
R¹ eine C₂₋₁₅-Gruppe, die eine ungesättigte und/oder cyclische Alkylgruppe sein kann, eine Aralkylgruppe oder eine ggfs. substituierte und/oder kondensierte aromatische und/oder heteroaromatische Gruppe,
R Wasserstoff, eine ggfs. substituierte geradkettige oder verzweigte oder cyclische und/oder ungesättigte C₁₋₇-Alkylgruppe oder eine aromatische Gruppe
und
R³ eine ggfs. substituierte geradkettige oder verzweigte oder cyclische und/oder ungesättigte C₁₋₇-Alkylgruppe oder eine aromatische Gruppe,
wobei R und R³ zusammen mit dem benachbarten Stickstoffatom einen 5- bis 8-gliedrigen Ring bilden können, der ggfs. ein oder mehrere Heteroatome enthält,
sowie ihrer Salze
durch
(A) Umsetzung eines Amidoxims der allgemeinen Formel II, 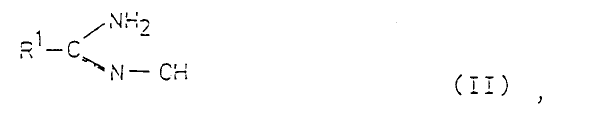
in der R¹ die oben definierte Bedeutung besitzt,
mit einem Alkalimetallhydroxid oder einem Alkalimetallalkoxid in Gegenwart eines organischen Lösungsmittels,
(B) Umsetzung des in Stufe A erhaltenen Amidoxim-Komplexes
(B1) mit einem Amin der allgemeinen Formel III 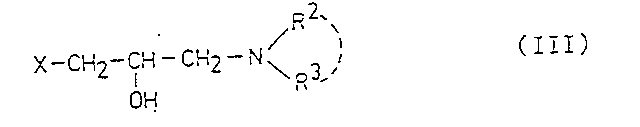
(B2) und/oder einem Amin der allgemeinen Formel IV, 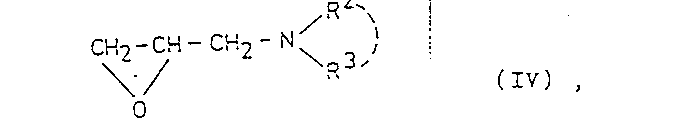 in der R und R³ die oben definierte Bedeutung besitzen und X ein Halogenatom darstellt,
unter SN2-Reaktionsbedingungen bei einer Temperatur im Bereich von 0 bis 100 °C
und
(C) Isolierung des erhaltenen Amidoxim-Derivats der Formel I
(D) sowie gegebenenfalls
Umwandlung des Amidoxim-Derivats der Formel I mit einer oder mehreren Säuren in das entsprechende Salz bzw. entsprechende Salzgemische sowie ggfs. Umwandlung der Salze in die freie Base und ggfs. Umwandlung der freien Base mit einer anderen Säure in das entsprechende Salz,
dadurch gekennzeichnet, daß
in Stufe A als organisches Lösungsmittel Dimethylformamid oder 1,3-Dimethyl-2-imidazolidinon eingesetzt werden.

2. Verfahren zur Herstellung von Amidoxim-Derivaten der allgemeinen Formel I, 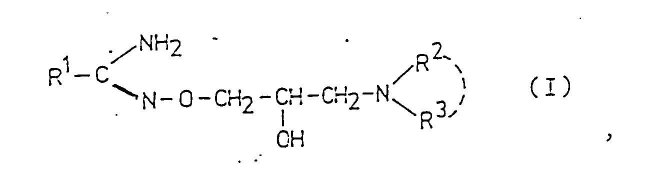 in der bedeuten:
R¹ eine C₂₋₁₅-Gruppe, die eine ungesättigte und/oder cyclische Alkylgruppe sein kann, eine Aralkylgruppe oder eine ggfs. substituierte und/oder kondensierte aromatische und/oder heteroaromatische Gruppe,
R Wasserstoff, eine ggfs. substituierte geradkettige oder verzweigte oder cyclische und/oder ungesättigte C₁₋₇-Alkylgruppe oder eine aromatische Gruppe
und
R³ eine ggfs. substituierte geradkettige oder verzweigte oder cyclische und/oder ungesättigte C₁₋₇-Alkylgruppe oder eine aromatische Gruppe,
wobei R und R³ zusammen mit dem benachbarten Stickstoffatom einen 5- bis 8-gliedrigen Ring bilden können, der ggfs. ein oder mehrere Heteroatome enthält,
sowie ihrer Salze
durch
(A) Umsetzung eines Amidoxims der allgemeinen Formel II, 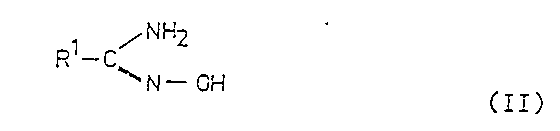
in der R¹ die oben definierte Bedeutung besitzt,
mit einem Alkalimetallhydroxid oder einem Alkalimetallalkoxid in Gegenwart eines organischen Lösungsmittels,
(B) Umsetzung des in Stufe A erhaltenen Amidoxim-Komplexes mit einer Aminoverbindung unter SN2-Reaktionsbedingungen bei einer Temperatur im Bereich von 0 bis 100 °C
und
(C) Isolierung des erhaltenen Amidoxim-Derivats der Formel I
(D) sowie gegebenenfalls
Umwandlung des Amidoxim-Derivats der Formel I mit einer oder mehreren Säuren in das entsprechende Salz bzw. entsprechende Salzgemische sowie ggfs. Umwandlung der Salze in die freie Base und ggfs. Umwandlung der freien Base mit einer anderen Säure in das entsprechende Salz,
dadurch gekennzeichnet, daß
- in Stufe A als organisches Lösungsmittel Dimethylformamid oder 1,3-Dimethyl-2-imidazolidinon
und
- in Stufe B als Aminoverbindung eine Verbindung der Formel V, 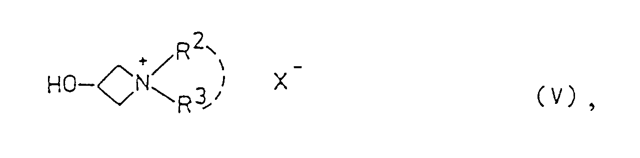 in der R und R³ die oben definierte Bedeutung besitzen und X ein Halogenatom darstellt,
eingesetzt werden.

3. Verfahren nach Anspruch 1 oder 2, dadurch gekennzeichnet, daß die Umsetzung in Stufe A in Gegenwart einer Protonenquelle durchgeführt wird.

4. Verfahren nach Anspruch 3, dadurch gekennzeichnet, daß in Stufe A als Protonenquelle t-Butanol verwendet wird.

5. Verfahren nach einem der Ansprüche 1 bis 4, dadurch gekennzeichnet, daß der in Stufe A erhaltene Amidoxim-Komplex ohne Isolierung in Stufe B eingesetzt wird.

6. Verfahren nach einem der Ansprüche 1 bis 5, dadurch gekennzeichnet, daß die Umsetzung des Amidoxim-Komplexes mit dem Amin in Stufe B bei einer Temperatur von 30 bis 75 °C vorgenommen wird.

7. Verfahren nach einem der Ansprüche 1 bis 6, dadurch gekennzeichnet, daß die Umsetzung des Amidoxim-Komplexes mit dem Amin in Stufe B in Gegenwart eines Metallsalzkatalysators durchgeführt wird.

8. Verfahren nach Anspruch 7, dadurch gekennzeichnet, daß als Metallsalzkatalysatoren Aluminiumoxid, Zinnoxid, Dibutylzinnoxid oder organische Komplexe dieser Verbindungen eingesetzt werden.

9. Verfahren nach einem der Ansprüche 1 bis 8, dadurch gekennzeichnet, daß das Amidoxim-Derivat der Formel I selektiv von Nebenprodukten getrennt wird.

10. Verfahren nach Anspruch 9, gekennzeichnet durch folgende Schritte zur selektiven Abtrennung des Amidoxim-Derivats der Formel I:
- Neutralisation des Reaktionsgemisches mit einer Säure bei einer Temperatur von 40 bis 70 °C,
gegebenenfalls nach Verdünnen des Reaktionsgemisches mit einem Lösungsmittel und/oder nach Filtration,
- Ansäuern des erhaltenen Gemisches,
- Einstellen eines pH-Werts von 4 bis 5 bei 50 °C oder eines pH-Werts von 2 bis 3 bei 70 °C und Abtrennung der ausgefällten Salze und Verunreinigungen aus dem Gemisch
und
- Kristallisation des mit einer Säure gebildeten Salzes des Amidoxim-Derivats der Formel I durch Einstellung des pH-Werts des Gemisches auf einen Wert von 1 bis 3.

11. Verfahren nach einem der Ansprüche 3 bis 10, dadurch gekennzeichnet, daß das Amin der Formel IV durch Umsetzung von Epichlorhydrin der Formel VI 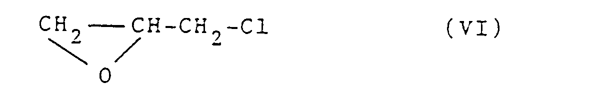 mit einem Amin der allgemeinen Formel VII, 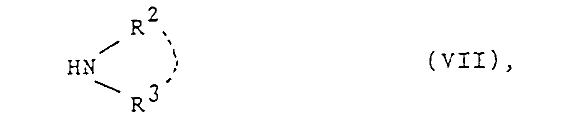 in der R und R³ die oben definierte Bedeutung besitzen,
in Gegenwart von t-Butanol in einem Massenverhältnis von t-Butanol zu Amin der Formel VII von 1:0,8 bis 1:1,2 hergestellt wird.

## Revendications

1. Un procédé de préparation de dérivés d'amidoxime de formule générale I, 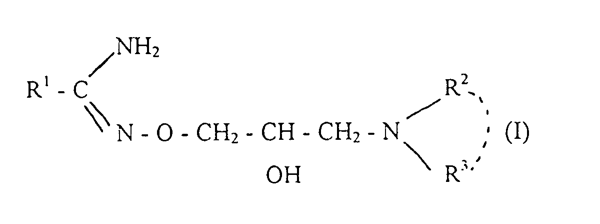 où :
R¹ est un groupe en C_{2-15,} qui peut être un groupe alkyle cyclique et/ou insaturé ou un groupe aralkyle ou un groupe aromatique et/ou hétéroaromatique condensé et/ou optionnellement substitué,
R est un hydrogène, un groupe alkyle en C₁₋₇ insaturé et/ou cyclique, à chaîne linéaire ou ramifiée, optionnellement substitué ou un groupe aromatique,
R³ est un groupe alkyle en C₁₋₇ insaturé et/ou cyclique, à chaîne linéaire ou ramifiée, optionnellement substitué ou un groupe aromatique, où
R et R³ peuvent ensemble former avec l'atome d'azote adjacent un cycle à 5 à 8 membres contenant optionnellement un ou plusieurs autres hétéroatomes,
et de leurs sels
par
(A) réaction d'un amidoxime de formule générale II, 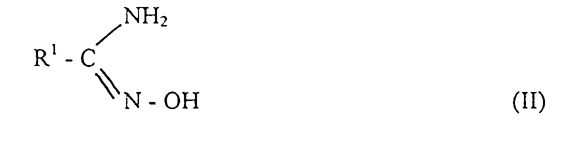
où R1 est tel que défini précédemment,
avec un hydroxyde de métal alcalin ou un alkoxyde de métal alcalin en présence d'un solvant organique,
(B) réaction du complexe d'amidoxime obtenu en étape (A)
(B1) avec une amine
de formule générale III 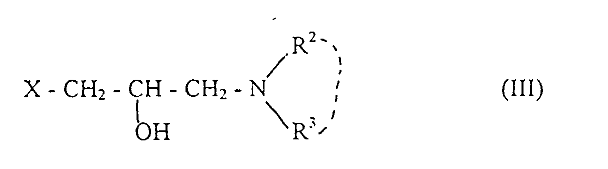
(B2) et/ou une amine
de formule générale IV 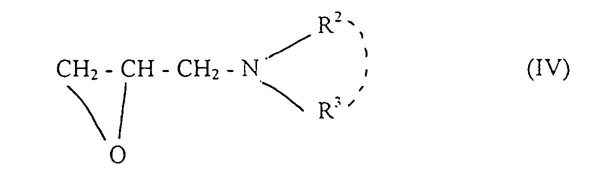
où R et R³ sont tels que définis précédemment, et X est un atome d'halogène,
dans des conditions de réaction sous SN₂ à une température comprise entre 0
et 100° C, et
(C) en isolant le dérivé d'amidoxime obtenu, de formule I
(D) et optionnellement
en convertissant le dérivé d'amidoxime de formule I avec un ou plusieurs acides respectivement en sel ou mélanges de sel correspondants, et en convertissant optionnellement les sels en base libre, puis en convertissant optionnellement la base libre avec un autre acide en sel correspondant,
caractérisé en ce que on utilise du diméthylformamide ou de la 1,3-diméthyl-2-imidazolidinone en tant que solvant organique à l'Etape A.

2. Un procédé de préparation de dérivés d'amidoxime de formule générale I, 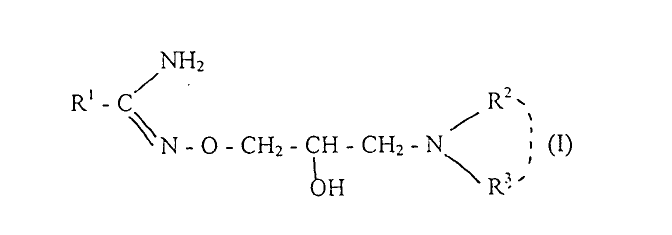 où :
R¹ est un groupe en C₂₋₁₅, qui peut être un groupe alkyle cyclique et/ou insaturé ou un groupe aralkyle ou un groupe aromatique et/ou hétéroaromatique condensé et/ou optionnellement substitué,
R est un hydrogène, un groupe alkyle en C₁₋₇ insaturé et/ou cyclique, à chaîne linéaire ou ramifiée, optionnellement substitué ou un groupe aromatique,
R³ est un groupe alkyle en C₁₋₇ insaturé et/ou cyclique, à chaîne linéaire ou ramifiée, optionnellement substitué ou un groupe aromatique, où
R et R³peuvent ensemble former avec l'atome d'azote adjacent un cycle à 5 à 8 membres contenant optionnellement un ou plusieurs autres hétéroatomes,
et de leurs sels
par
(A) réaction d'un amidoxime de formule générale II, 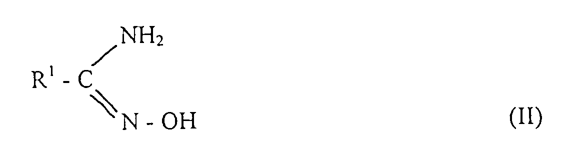
où R¹ est tel que défini précédemment,
avec un hydroxyde de métal alcalin ou un alkoxyde de métal alcalin en présence d'un solvant organique,
(B) réaction du complexe d'amidoxime obtenu en étape A avec un composé amino dans des conditions de réaction sous SN₂ à une température comprise entre 0 et 100° C,
et
(C) en isolant le dérivé d'amidoxime obtenu, de formule I
(D) et optionnellement
en convertissant le dérivé d'amidoxime de formule I avec un ou plusieurs acides respectivement en sel ou mélanges de sel correspondants, et en convertissant optionnellement les sels en base libre, puis en convertissant optionnellement la base libre avec un autre acide en sel correspondant,
caractérisé en ce que
- on utilise du diméthylformamide ou de la 1,3-diméthyl-2-imidazolidinone en tant que solvant organique à l'Etape A,
et
- on utilise en tant que composé amino à l'Etape B un composé de formule V, 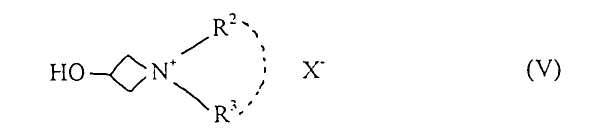 où R et R³ sont tels que définis précédemment, et X est un atome d'halogène.

3. Le procédé selon la Revendication 1 ou 2, caractérisé en ce que la réaction de l'Etape A est effectuée en présence d'une source de proton.

4. Le procédé selon la Revendication 3, caractérisé en ce que du butanol tertiaire est utilisé en tant que source de proton à l'Etape A.

5. Le procédé selon l'une des Revendications 1 à 4, caractérisé en ce que le complexe d'amidoxime obtenu à l'Etape A est utilisé à l'Etape B sans isolation.

6. Le procédé selon l'une des Revendications 1 à 5, caractérisé en ce que la réaction du complexe d'amidoxime avec l'amine en Etape B est effectuée à une température comprise entre 30 et 75° C.

7. Le procédé selon l'une des Revendications 1 à 6, caractérisé en ce que la réaction du complexe d'amidoxime avec l'amine en Etape B est effectuée en présence d'un catalyseur de sel métallique.

8. Le procédé selon la Revendication 7, caractérisé en ce qu'on utilise de l'oxyde d'aluminium, de l'oxyde d'étain, de l'oxyde de dibutylétain ou des complexes organiques de ceux-ci en tant que catalyseurs de sel métallique.

9. Le procédé selon l'une des Revendications 1 à 8, caractérisé en ce que le dérivé d'amidoxime de formule I est séparé de façon sélective des produits dérivés.

10. Le procédé selon la Revendication 9, caractérisé par les étapes suivantes de séparation sélective du dérivé d'amidoxime de formule I :
- neutralisation du mélange de réaction avec un acide à une température comprise entre 40 et 70° C,
optionnellement après dilution du mélange de réaction avec un solvant et/ou après filtration,
- acidification du mélange obtenu,
- ajustement d'une valeur de pH comprise entre 4 et 5 à 50° C ou d'une valeur de pH comprise entre 2 et 3 à 70° C, et séparation des sels précipités et contaminants du mélange,
et
- cristallisation du sel du dérivé d'amidoxime de formule I formé avec un acide en ajustant la valeur de pH du mélange entre 1 et 3.

11. Le procédé selon l'une des Revendications 3 à 10, caractérisé en ce que l'amine de Formule IV est préparée par réaction d'épichlorohydrine de Formule VI 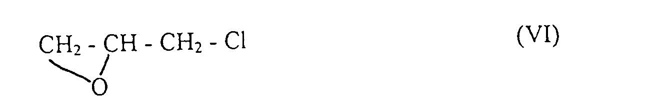 avec une amine de formule générale VII, 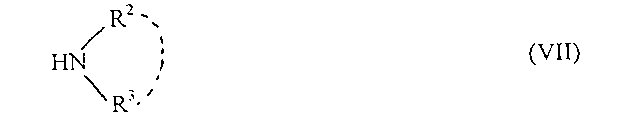
où R et R³ sont tels que définis dans la Revendication 1,
en présence de butanol tertiaire dans une proportion de masse entre butanol tertiaire et amine de Formule VII comprise entre 1:0,8 et 1:1,2.
